# EUROPEAN PATENT APPLICATION

(11) **EP 0 630 630 A2**
(43) Date of publication of application: **28.12.1994**
(21) Application number: 94304542.7
(22) Date of filing: 22.06.1994
(51) Int. Cl.: A61F 13/15

(54) **Disposable undergarment**

(30) Priority: 22.06.1993 JP 33608/93
(71) Applicant: UNI-CHARM CORPORATION, Kawanoe-shi Ehime-ken (JP)
(72) Inventor: Yamamoto, Masamitsu, Kawanoe-shi, Ehime-ken (JP); Fujioka, Yoshihisa, Mitoyo-gun, Kagawa-ken (JP); Ono, Yoshio, Kawanoe-shi, Ehime-ken (JP); Kaji, Miwako, Kawanoe-shi, Ehime-ken (JP)
(74) Representative: Warren, Anthony Robert

(57) **Abstract**

A disposable undergarment comprises a basic cloth (1) made of fibrous nonwoven fabric elastically stretchable in length and width and provided with a waist-opening (6) and leg-openings (7). A fluidized elastic material, which has rubber-like elasticity when cured, is applied to the leg-openings along their peripheral surfaces fully to the outer peripheral edges thereof to form ribbon-like elastic zones (9).

## Description

The present invention relates to a disposable undergarment of the pants type formed by bonding front and rear bodies along respective opposite side edges at waist level, such as incontinence diapers, baby diapers, baby training pants and sanitary pants.

Such a disposable undergarment of the prior art usually employs non-elastic nonwoven fabric as a base cloth and elastic members each comprising a plurality of thread-like elastic elements or a single tape-like elastic member bonded, after being stretched by a predetermined elongation percentage, to respective leg-openings along peripheral edges thereof by means of hot melt type adhesive in order to keep the leg-openings fitting around the wearer's legs and thereby to avoid excretion leakage possibly occurring around the legs.

However, some of the machines for high speed production of undergarments are unable to attach said elastic members along outer peripheral edges of the respective leg-openings and therefore it is forced to attach them to the leg-openings along regions inwardly spaced from the respective outer peripheral edges. Consequently, there are regions having no elastic members between the respective elastic members attached in this manner and the outer peripheral edges of the respective leg-openings and these regions correspond to a material loss for undergarment. In addition, these regions may prevent neatly wearing the undergarment and cause leakage of excretion, particularly urine from between these regions and the wearer's skin because said regions are apt to be bent backward or disengaged from the wearer's skin. Furthermore, separately of the elastic members, adhesive must be applied to the leg-openings or the elastic members, so the labor cost as well as the material cost are correspondingly increased.

It is an object of the invention to eliminate the above-mentioned problems by applying, instead of the elastic members, fluidized elastic material having rubber-like elasticity in its cured state to said openings to form ribbon-like elastic zones.

The object set forth above is achieved, according to the invention, by a disposable undergarment comprising a basic cloth made of fibrous nonwoven fabric elastically stretchable at least in length and width and provided with a waist-opening and a pair of leg-openings, characterized in that fluidized elastic material having rubber-like elasticity in its cured state is applied to said leg-openings along their peripheral surfaces almost fully to outer peripheral edges thereof to form ribbon-like elastic zones, respectively, and a stretching stress of the respective leg-openings along said ribbon-like elastic zones is adjusted to be higher than that in the remaining region of said undergarment.

Outer peripheral edges of the respective leg-openings tightly fit around wearer's legs and bending backward as well as disengagement of said outer peripheral edges from the wearer's skin during use can be effectively avoided.

The invention will be described by way of example with reference to the accompanying drawings, in which:
Fig. 1 is a front view showing a first embodiment of a disposable undergarment constructed according to the invention;
Fig. 2 is an enlarged scale sectional view taken along a line X-X in Fig. 1;
Fig. 3 is an enlarged scale sectional view taken along a line Y-Y in Fig. 1;
Fig. 4 is a front view showing a second embodiment of the invention;
Fig. 5 is a developed view showing, as partially broken away, the inside of the undergarment shown by Fig. 3; and
Fig. 6 is an enlarged scale sectional view taken along a line Z-Z in Fig. 5.

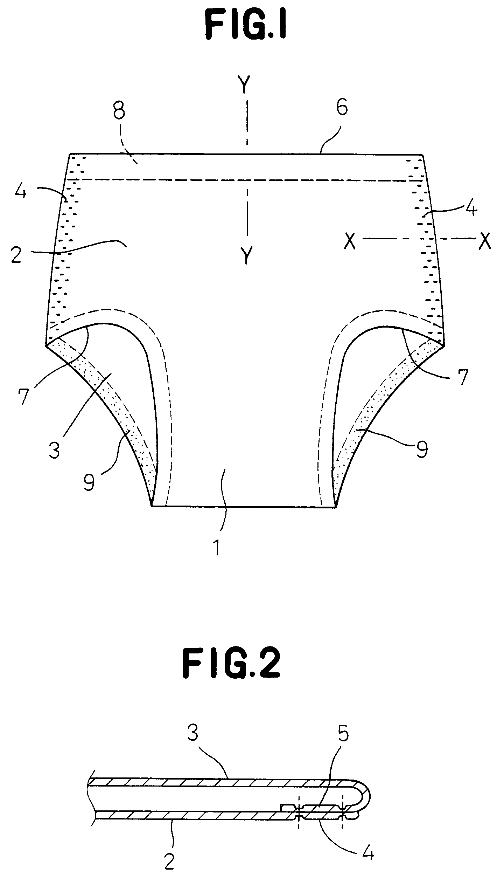

Referring to Figs. 1 through 3, there is shown a first embodiment of the invention. An undergarment is cut out from fibrous nonwoven fabric used as a basic cloth 1 having an elastic stretchability both in length and width (preferably, an elastic stretchability higher in width than in length) and has a waist-opening 6 and a pair of leg-openings 7 formed by bonding opposite side edges 4, 5 of a front body 2 to corresponding opposite side edges 4, 5 of a rear body 3 in dotted pattern.

The basic cloth 1 of the front and rear bodies 2, 3 may be formed, for example, from web of heat crimped synthetic fibre having its component fibres being entangled or intertwined by subjecting them to fluid jet or intermittently bonding together under pressure and heat.

The waist-opening 6 is provided on its inner periperal surface with an elastic member 8 in the form of tape so as to be elastically stretchable in a circumferential direction. The elastic member 8 is preferably of color different from that of the basic cloth 1 for the front and rear bodies 2, 3 because the elastic member 8 will be seen through said basic cloth and thereby an aethetic effect will be obtained.

Synthetic resin of well known hot melt type presenting rubber-like elasticity in its cured state, which has been conventionally employed for bonding members of a disposable diaper, is applied to the inner peripheral surface of each leg-opening 7 fully to the outer peripheral edge thereof to form a ribbon-like elastic zone 9 having a stretching stress of 50 to 130 g. As a result, a region of the leg-opening 7 occupied by the ribbon-like elastic zone 9 has a stretching stress higher than that in the remaining region of the basic cloth 1. Obviously, it is possible to make such comparative measurement of the stretching stress on the same-sized pieces of the respective regions cut off from the basic cloth 1.

It is also possible to use, instead of said resin, so-called rubber elastomer which is elastic material in the form of liquid or gel presenting a rubber-like elasticity in its cured state or after heat treatment, such as thermoplastic elastomers of styrenics, polyolefin, polyurethane, polyester, polyvinyl chloride and natural rubber.

To coat each leg-opening 7 fully to its outer peripheral edge with the fluidized elastic material, such as above-mentioned resin or rubber elastomer, the basic cloth 1 is coated, before the region to form each leg-opening 7 is cut off from the basic cloth 1, with said fluidized elastic material so that the ribbon-like elastic zone 9 may be defined over a surplus coated area and a line along which said region is to be cut may be located within said surplus coated area.

If it is apprehended that the ribbon-like elastic zone 9 formed on the inner peripheral surface of the leg-opening 7 might give the wearer's skin an uncomfortable feeling, at least the zone 9 may be covered with a ribbon-like nonwoven fabric of elastic fibre,or said zone 9 may be provided on the outer peripheral surface of the leg-opening 7.

The elastic material such as the previously mentioned resin used to form the ribbon-like elastic zone 9 may be selected from those having colors other than that of the basic cloth 1 for the front and rear bodies 2, 3 to provide the leg-opening 7 with a trimming pattern and thereby to obtain an aethetic effect just as in the case of the waist-opening 6.

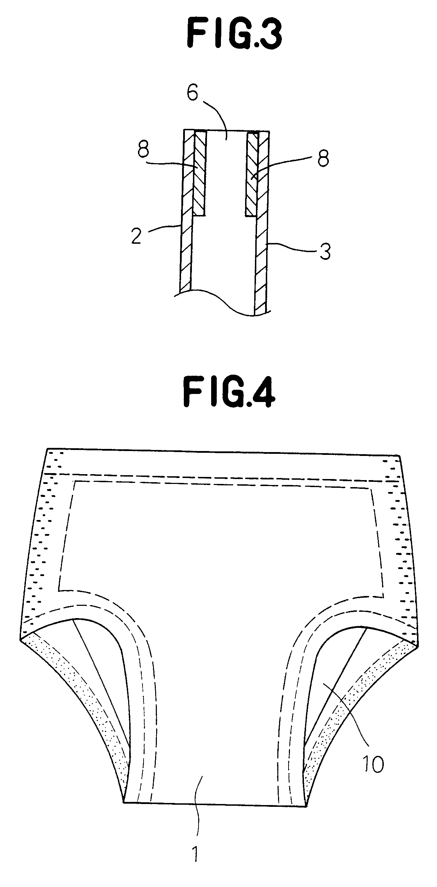

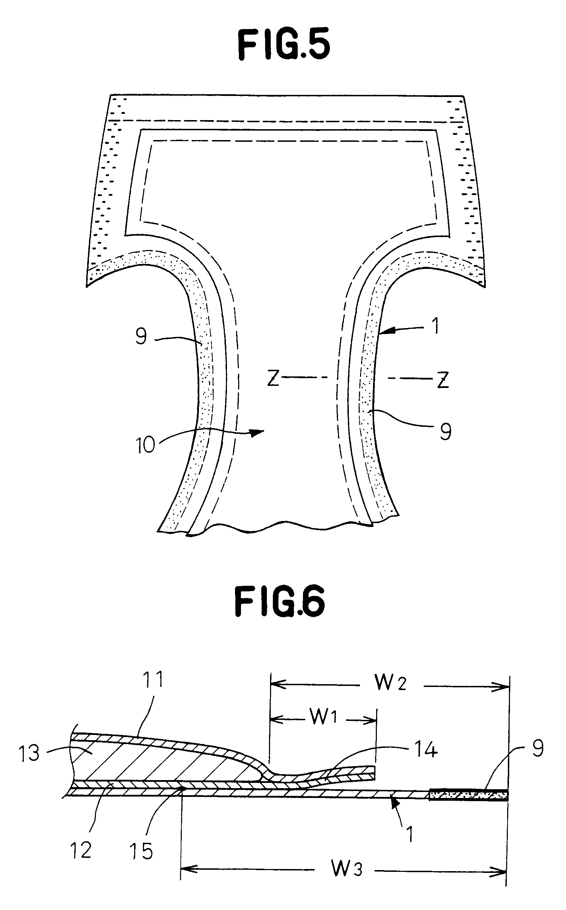

Referring to Figs. 4 through 6, there is shown a second embodiment of the invention. An absorbent pad 10 is laid upon the inner surface of the basic cloth 1. The absorbent pad 10 has a contour substantially similar to that of the basic cloth 1 and a size smaller than that of the basic cloth 1. It should be understood that the elastic stretchability of the absorbent pad 10, particularly of the liquid-absorbent core 13 may be lower than that of the basic cloth 1 to such a degree that the liquid-absorbent core 13 can be free from undesirable deformation such as collapse or breakage due to a stretch behavior of the basic cloth 1. Regions of the top- and backsheets extending outward from outer peripheral edges of the core 13 are bonded together by means of adhesive or suitable welding means to form side flaps 14, each having a width W₁, on opposite sides of the absorbent pad 10. The backsheet 12 may be formed, if desired, by a liquid-impermeable sheet such as water-proofed fibrous nonwoven fabric or plastic film.

The core 13 is so shaped to have a thickness gradually decreasing from a region adjacent its outer peripheral edges to its outer peripheral edges,so that an abrupt difference in level which otherwise would appear between the basic cloth 1 and the outer peripheral edges of the absorbent pad 10 may be avoided and, in consequence, a zone adjacent each leg-opening 7 may fit well around the wearer's leg. Such a desired shape of the core 13 can be achieved, for example, be appropriate allotment of core materials. The absorbent pad 10 is intermittently (preferably in dotted pattern) bonded to the basic cloth 1 by means of adhesive or suitable welding means (not shown). A distance (width) W₂ from the inner side edge of each side flap 14 to the corresponding outer side edge of the basic cloth 1 depends on the rigidity of the absorbent pad 10 and is preferably 10mm or larger to maintain a desired contractile force of the ribbon-like elastic zone 9. For the same reason and to maintain an area of the basic cloth that can be elastically stretched independently of the absorbent pad 10 as large as possible, it is preferred that each outermost side edge 15 of the region over which the absorbent pad 10 is bonded to the basic cloth 1 is located inside the inner side edge of the corresponding side flap 14 and a distance W₃ from each outermost side edge 15 to the corresponding outer side edge of the basic cloth 1 is 15 mm or larger.

With the undergarment constructed as described hereinabove according to the invention, the outer peripheral edges of the respective leg-openings tightly fit around the wearer's legs, so a bending backward as well as a disengagement of said outer peripheral edges from the wearer's skin during use can be effectively avoided. Accordingly, all the drawbacks of the conventional undergarment,such as excretion leakage and difficulty to wear the undergarment neatly,can be eliminated.

## Claims

1. A disposable undergarment comprising a basic cloth made of fibrous nonwoven fabric,elastically stretchable at least in length and width,and provided with a waist-opening and a pair of leg-openings, characterized in that fluidized elastic material,having a rubber-like elasticity in its cured state,is applied to said leg-openings along their peripheral surfaces to or almost fully to the outer peripheral edges thereof to form ribbon-like elastic zones, and a stretching stress of the respective leg-openings along said ribbon-like elastic zones is adjusted to be higher than that in the remaining region of the undergarment.

2. A disposable undergarment according to Claim 1,
said undergarment further comprising an absorbent pad having a size smaller than that of said basic cloth and being laid upon the inner surface of said basic cloth so that outer peripheral edges of said basic cloth may extend outward from those of said absorbent pad.

3. A disposable undergarment according to Claim 2, wherein said absorbent pad is shaped to have a thickness gradually decreasing from a region adjacent its opposite side edges to its opposite side edges.

4. A disposable undergarment according to Claim 2 or 3, wherein said absorbent pad is intermittently bonded to said basic cloth,while its opposite side edges are not bonded to said basic cloth.
